(19) Europäisches Patentamt
European Patent Office
Office européen des brevets

(11) Publication number: 0 098 625 A1

(12) # EUROPEAN PATENT APPLICATION

(21) Application number: 83200775.1

(51) Int. Cl.[3]: C 07 C 103/50

(22) Date of filing: 02.06.83

(30) Priority: 11.06.82 US 387565

(43) Date of publication of application: 18.01.84 Bulletin 84/3

(84) Designated Contracting States: BE CH DE FR GB IT LI NL

(71) Applicant: THE PROCTER & GAMBLE COMPANY, 301 East Sixth Street, Cincinnati Ohio 45202 (US)

(72) Inventor: Reitz, David Bruce, 1702 Shallowbrook Lane, St. Louis Missouri 63141 (US)

(74) Representative: Suslic, Lydia et al, Procter & Gamble European Technical Center Temselaan 100, B-1820 Strombeek-Bever (BE)

(54) **Process for preparing alpha-aspartic acid amides.**

(57) A novel process for preparing alpha-aspartic acid amides, particularly those suitable as non-nutritive sweeteners. This process involves the coupling reaction of an alpha-aspartate ester and a beta-hydroxy primary amine. The ester is reacted with the amine, one of which is preferably in stoichiometric excess, in a liquid phase system to form the alpha-aspartic acid amide. The amide is then recovered from the liquid phase system.

EP 0 098 625 A1

DAVID B. REITZ 0098625

## TECHNICAL FIELD

The present application relates to the preparation of alpha-aspartic acid amides, in particular those amides suitable as non-nutritive sweeteners.

A number of alpha-aspartic acid amides have been found to be suitable as non-nutritive sweeteners. The most prominent examples are the alpha-L-aspartyl-L-phenylalanine lower alkyl esters, in particular the methyl ester. Because these L-phenylalanine esters have hydrolytic stability problems, the art has searched for alternative aspartic acid amides having a non-nutritive sweetening capacity. One such example is the 1-hydroxymethyl aspartic acid amide sweeteners disclosed in U.S. Patent n° 4,338,346, granted on July 6, 1982 ——————— and which have the following formula:

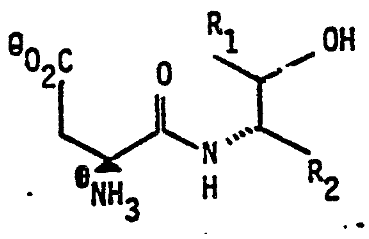

wherein $R_1$ is hydrogen and $R_2$ is a $C_4$-$C_9$ alkyl group, the amide having an L,L (S,S) configuration. Another such example is the 1-hydroxyethyl aspartic acid amide sweeteners disclosed in EPO Patent Application 0 068 551, published on January 5, 1983 and having the following formula:

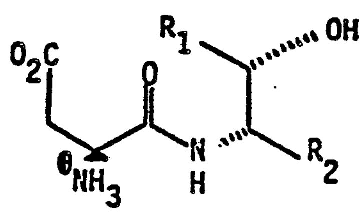

wherein $R_1$ is methyl and $R_2$ is a $C_4$-$C_9$ alkyl group, the amide having an (S,S,S) configuration.

The amides of the Brand and Rizzi Applications are formed by coupling certain beta-hydroxy primary amines (i.e., 2-aminoalcohols) with an alpha-L-aspartyl moiety. While the economical

synthesis of the beta-hydroxy primary amines is particularly important, attention should also be given to the coupling reaction. The coupling reaction desirably involves an uncomplicated, inexpensive synthesis step which is regiospecific for formation of the sweet alpha-aspartic acid amides as opposed to the non-sweet beta-aspartic acid amides. The coupling reaction should also provide a relatively high yield of the particular amide at a relatively fast reaction rate. Further, retention of the specific stereochemistry at the asymmetric carbon centers is particularly critical during this coupling reaction.

One method for preparing the amides of the Brand and Rizzi Applications involves the coupling of the beta-hydroxy primary amines with an aspartic acid reagent having protecting groups. One such reagent is N-carbobenzyloxy-L-aspartic acid, in particular the beta-benzyl ester thereof. One disadvantage of this synthesis is the additional step(s) required to remove the protecting groups. Also, these reagents having protecting groups are fairly expensive. Further, coupling reactions involving such protecting group reagents require special coupling agents such as dicyclohexylcarbodiimide or a combination of N-methylmorpholine and isobutylchloroformate.

Another method for coupling the beta-hydroxy primary amine to the aspartyl moiety involves activated aspartic acid reagents, in particular the aspartic anhydrides. Examples of such reagents include the hydrohalide salts of aspartic anhydride, as well as N-trifluoroacetyl or N-formyl aspartic anhydride. The direct reaction of these aspartic anhydrides with the beta-hydroxy primary amines is not regiospecific. Instead, mixtures of alpha- and beta-coupled amides are formed. Generally, the alpha form predominates in a ratio of from 3:1 to 4:1.

It is therefore an object of the present invention to prepare alpha-aspartic acid amides by an uncomplicated, inexpensive coupling synthesis.

It is another object of the present invention to provide a coupling synthesis for alpha-aspartic acid amides which is regiospecific for the alpha-, as opposed to the beta-, amides.

It is yet another object of the present invention to prepare

alpha-aspartic acid amides in high yield.

It is a further object of the present invention to prepare alpha-aspartic acid amides by a method having a relatively fast reaction rate.

It is yet a further object of the present invention to prepare alpha-aspartic acid amides wherein the specific stereochemistry of the amide is retained during the coupling reaction.

These and other objects of the present invention are hereinafter disclosed.

## OTHER BACKGROUND ART

Kovacs et al., "Chemical Studies of Polyaspartic Acids", J. Org. Chem., Vol. 26, (1961), pp. 1084-91, discloses the preparation of methyl alpha-L-aspartate by reacting methanol with the hydrobromide salt of aspartic anhydride. The homogeneity of this aspartate ester was established by conversion thereof to L-isoasparagine with ammonia. See also Kovacs et al., "Glutamic and Aspartic Anhydrides: Rearrangement of N-carboxyglutamic 1,5-anhydride to the Leuchs' Anhydride and Conversion of the Latter to Pyroglutamic Acid, J. Am. Chem. Soc., Vol. 85, (1963), pp. 1839-44, which also discloses the reaction of methyl alpha-L-aspartate with liquid ammonia to provide L-isoasparagine.

M. Bodanszky et al., Peptide Synthesis, (2nd Ed. 1976), pp. 99-106, discloses a peptide synthesis scheme involving an aminolysis reaction using activated esters of the amino acids. The amino group of one of the amino acids typically has a protecting group. An example of such reactions is as follows:

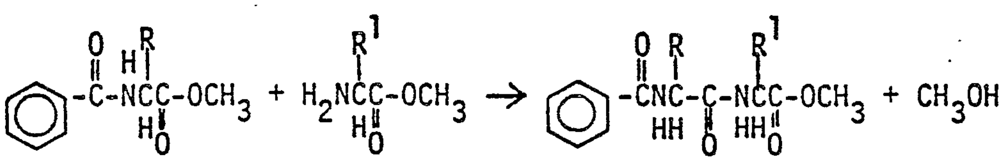

U.S. Patent 3,962,207 to Uchiyama et al., issued June 8, 1976 discloses the preparation of alpha-L-aspartyl-L-phenylalanine esters by the reaction of the hydrohalide salt of aspartic anhydride with

the L-phenylalanine ester in the presence of an alcohol (e.g., methanol) and a strong acid (e.g., sulfuric acid). The strong acid and alcohol can be added to a solution of the anhydride salt. The L-phenylalanine ester is then added to this solution and mixed. See also U.S. Patent 3,833,553 to Ariyoshi et al., issued September 3, 1974, which discloses the preparation of alpha-L-aspartyl-L-phenylalanine esters by reacting the L-phenylalanine ester with a salt of aspartic anhydride in the presence of a weak acid such as acetic acid; the anhydride can be dissolved in an inert liquid medium (e.g., methanol) to which is admixed the phenylalanine ester and the weak acid.

## DISCLOSURE OF THE INVENTION

The present application relates to a novel process for preparing alpha-aspartic acid amides, in particular those disclosed as non-nutritive sweeteners in the Brand and Rizzi Applications. This process involves the coupling reaction of an alpha-aspartate ester with a beta-hydroxy primary amine. The ester is reacted with the amine, one of which is preferably in stoichiometric excess, in a liquid phase system to form the alpha-aspartic acid amide. The amide is then recovered from the liquid phase system.

The process of the present application provides basically a single step, inexpensive synthesis for preparing the desired alpha-aspartic acid amides. The alpha-amide is formed almost exclusively by this process. A high yield of the amide can be provided at a relatively fast reaction rate. The stereochemistry of the coupled amide is also retained by this process.

## DETAILED DESCRIPTION OF THE INVENTION

### A. Alpha-Aspartate Esters and Beta-Hydroxy Primary Amines.

A number of different alpha-aspartate esters can be used in the process of the present application. In preparing non-nutritive sweeteners, the esters are preferably the alpha-L-aspartate esters. Examples of such esters include methyl alpha-L-aspartate, ethyl

alpha-L-aspartate, benzyl alpha-L-aspartate and cellosolve esters of alpha-L-aspartate (e.g., the 2-methoxyethyl ester). These esters can be prepared by reacting an aspartic anhydride derivative with the respective alcohol. See Kovacs et al., "Chemical Studies of Polyaspartic Acids," J. Org. Chem., Vol. 26, (1961), p. 1089 (herein incorporated by reference), and Kovacs et al., "Glutamic and Aspartic Anhydrides: Rearrangement of N-carboxyglutamic 1,5-anhydride to the Leuchs' Anhydride and Conversion of the Latter to Pyroglutamic Acid," J. Am. Chem. Soc., Vol. 85, (1963), pp. 1843-44 (herein incorporated by reference), which disclose methods for preparing methyl, ethyl and benzyl alpha-L-aspartate esters.

A variety of beta-hydroxy primary amines can also be used in the process of the present application. These amines have the following general formula:

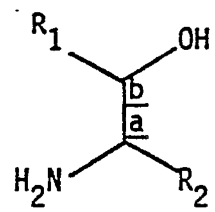

In preparing non-nutritive sweeteners, $R_1$ is preferably hydrogen or methyl and $R_2$ is preferably a $C_4$-$C_9$ alkyl group (e.g., straight or preferably branched chain alkyl, or cycloalkyl). $R_2$ is most preferably 3-methylbutyl. The asymmetric carbon centers at a and b also preferably have an (S) configuration. These preferred amines can be prepared by the methods disclosed in U.S. Application Serial No. 178,231 to L. M. Brand, filed August 14, 1980 (herein incorporated by reference), and EPO Patent Application 0 068 551, published on January 5, 1983 ——————— (herein incorporated by reference).

B. Liquid Phase System.

In the process of the present application, the alpha-aspartate ester and the beta-hydroxy primary amine are reacted in a liquid phase system. One example of such a liquid phase system involves a melt-type reaction wherein the amine and ester are heated and melted together. The preferred liquid phase system is a solvent system. This solvent system can comprise either a non-polar or polar

solvent. Suitable solvents include dioxane, tetrahydrofuran, acetonitrile, the $C_1$-$C_3$ alcohols, 1,2-dimethoxyethane, ethylene glycol and glycerol. A particularly preferred solvent is methyl alcohol.

The alpha-aspartate ester is preferably at least partially dissolved in the solvent system with the beta-hydroxy primary amine being subsequently added. The amine or the ester is preferably in a stoichiometric excess with the excess reagent depending upon the amine involved. For the 2-aminoalcohols ($R_1$ = hydrogen), either the ester or the amine can be in excess. For the 1-methyl-2-aminoalcohols ($R_1$ = methyl), the amine is preferably in excess for a faster reaction rate and higher yield of the amide. Typically, the mole ratio of the excess reagent to the other is at least about 2:1, and preferably at least about 4:1. The concentration of the excess reagent is typically at least about 0.2 M while the concentration of the other reagent is typically at least about 0.1 M. Preferably, the concentration of the excess reagent is at least about 2 M while the concentration of the other reagent is at least about 1 M. A higher mole ratio and/or a higher concentration of both the ester and the amine increases the reaction rate with a higher yield of the alpha-aspartic acid amide.

C. Reaction Conditions.

The alpha-aspartate ester is reacted with the beta-hydroxy primary amine at a temperature effective to cause formation of the respective alpha-aspartic acid amide in an appropriate yield and at a sufficiently fast reaction rate. Typically, the reaction temperature is at least about 40°C, and preferably within the range of from about 50° to about 100°C. In preparing the 1-hydroxyethyl aspartic acid amides of the Rizzi Application, (i.e., where $R_1$ is methyl for the beta-hydroxy primary amine), the reaction temperature preferably does not exceed about 60°C. At temperatures higher than about 60°C, there is a tendency for the asymmetric carbon center of the aspartyl moiety of the amide to lose its (S) stereochemical configuration.

The solvent used to dissolve the alpha-aspartate ester is

preferably removed from the solvent system during the reaction to improve the yield of the amide and to increase the reaction rate. Typically, the solvent is a highly volatile compound (e.g., methyl or ethyl alcohol). In such cases, the solvent can be removed by subjecting the solvent system to an appropriate vacuum. It is believed that removal of the solvent during the course of the reaction encourages crystallization and subsequent precipitation of the desired amide from the solvent system.

D. Recovery of the Amide from the Liquid Phase System.

The alpha-aspartic acid amide formed is preferably recovered from the liquid phase system during the reaction to improve the yield and increase the reaction rate. A preferred method involves precipitating the amide from the liquid phase (solvent) system. In such a precipitation step, the solvent system is selected so that the amide formed is insoluble therein. Once the amide is recovered, it can be purified by appropriate art recognized techniques, such as solvent recrystallization.

Specific Synthesis of L-1-Hydroxymethyl Alpha-L-Aspartic Acid Amides.

The following is a general reaction scheme for synthesizing L-1-hydroxymethyl alpha-L-aspartic acid amides:

(1)
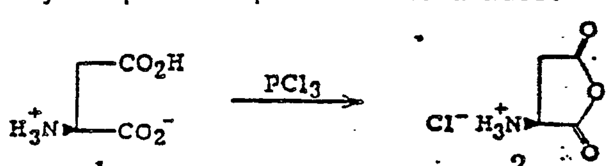


1 2

(2)
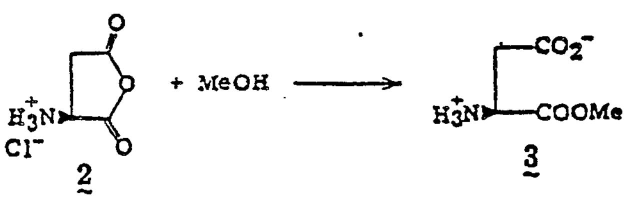


2 3

(3)
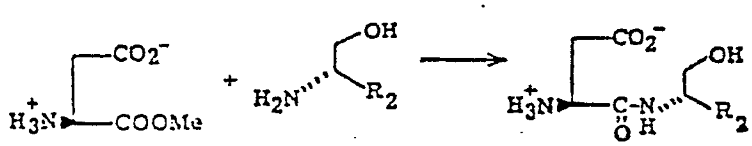

3 4 5

+ MeOH

In step (1) of the above scheme, L-aspartic acid 1 is converted by phosphorus trichloride to the hydrochloride salt of aspartic anhydride 2. In step (2), anhydride 2 is converted by methyl alcohol to methyl alpha-L-aspartate 3. In step (3), ester 3 is coupled with 2-aminoalcohol 4 to form amide 5 and methyl alcohol.

A. Amides Prepared From L-Leucinol and L-2-aminoisoheptanol.

The preparation of amides 5 from L-leucinol ($R_2$ = isobutyl) and L-2-aminoisoheptanol ($R_2$ = 3-methylbutyl) by the above reaction scheme is described hereafter:

Step (1): L-Aspartic Anhydride HCl.

200 g. (1.5 moles) of commercial L-aspartic acid was suspended in 1 l. of dry tetrahydrofuran (THF) and was stirred mechanically under a blanket of argon. This mixture was maintained at about room temperature with an external water bath while 70 ml. (0.8 moles) of reagent grade phosphorus trichloride was added dropwise over 15 minutes. This mixture was stirred for 17 hours at room temperature and then cooled and stirred for an additional 2.5 hours at 5°-10°C. The crude L-aspartic anhydride HCl was separated from the mixture by filtration and then washed with ice-cold THF under argon. After vacuum drying at room temperature, 233 g. of a yellow solid was obtained (m.p. 141°C). The identity of the anhydride was confirmed by its infrared spectrum.

Step (2) Methyl Alpha-L-Aspartate.

20.04 g. (0.132 moles) of the anhydride from step (1) was added to 100 ml of anhydrous methanol at 4°C and stirred at 4°C until solution was complete (15 min.). After warming to room temperature (22°C), 18.5 ml. of triethylamine was added and the reaction product was removed by filtration. Recrystallization of the crude product from aqueous ethanol gave 11.86 g. of methyl alpha-L-aspartate, m.p. 181-183.5°C, $\alpha]_D^{RT}$+ 35.87° (C, 1.408, $H_2O$), 61% yield; compared to literature values of m.p. 181-182°C and $\alpha]_D^{RT}$+ 43.3°. The optical purity of the ester was shown to be at least 98% based on

hydrolysis back to L-aspartic acid.

Step (3): N-(alpha-L-aspartyl)-L-leucinol.

A mixture containing 2.3 g. (0.020 moles) of L-leucinol, 1.47 g. (0.010 moles) of methyl alpha-L-aspartate from step (2), and 20 ml. of anhydrous methanol was stirred and heated under reflux for 4.5 hours. The methanol was removed under a vacuum and 200 ml. of ethyl ether was added to facilitate isolation of the crude product which was filtered off. Recrystallization of the crude product from 20 ml. of boiling water gave (after vacuum drying) 1.13 g. of glistening white platelets (54% yield). Product homogeneity and identity was established by TLC analysis and infrared spectra comparisons to a known sample of the amide.

Step (3): N-(alpha-L-aspartyl)-(2S)-2-aminoisoheptanol

A mixture containing 5.25 g. (0.040 moles) of (2S)-2-aminoisoheptanol, 1.47 g. (0.010 moles) of methyl alpha-L-aspartate from step (2), and 100 ml. of anhydrous methanol was stirred and heated under reflux for 4 hours with 88 ml. of the methanol being distilled off for 2 additional hours. Residual methanol was removed under a vacuum at 40°C. The residue was treated with 200 ml. of ethyl ether to precipitate the amide. Recrystallization of the amide from 100 ml. of boiling water gave (after vacuum drying) 1.47 g. of glistening crystals (m.p. 231°C, $\alpha]_D^{RT}$ + 13.7°) (67% yield). The identity of the amide was confirmed by comparison of its infrared spectrum and TLC behavior with the amide prepared by a different method.

B. Preparation of Amides from Other 2-Aminoalcohols.

Amides were prepared from other 2-aminoalcohols using the above reaction scheme. The results are presented in the following Table:

TABLE I

| Amino Alcohol | Alpha-Amide Yield (%) | m.p. (°C) | $\alpha]_D^{RT}$** |
|---|---|---|---|
| 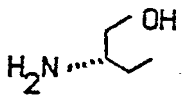 | 64 | 199-200 | +35.06° |
| 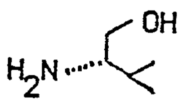 | 39 | 197-204.5 | +8.25° |
| 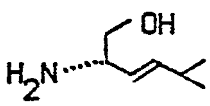 | 34 | 207-217 | E/Z Mixture |
| 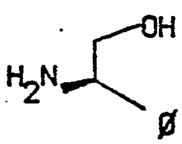 * | 44 | 235-238 | -62.87° |
| 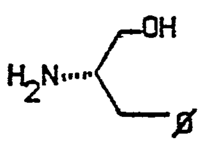 | 58 | 229.5-230 | -29.5° (AcOH) |

*(2R)-2-aminoalcohol

** Solvent was 0.5 N HCl, except where noted; RT = ca. 22°C ± 2°C.

Specific Synthesis of 1-Hydroxyethyl Alpha-L-Aspartic Acid Amides

The general reaction scheme for synthesizing 1-hydroxymethyl alpha-L-aspartic acid amides can be used for preparing 1-hydroxyethyl alpha-L-aspartic acid amides. The preparation of such an amide from (3S,2S)-3-amino-2-hydroxy-6-methylheptane ($R_2$ = 3-methylbutyl) is as follows:

Step (3):

N-(alpha-L-Aspartyl)-(3S,2S)-3-amino-2-hydroxy-6-methylheptane

A mixture of 0.135 g. of methyl alpha-L-aspartate from step (2), 0.425 g. of (3S,2S)-3-amino-2-hydroxy-6-methylheptane and 2.0 ml of acetonitrile was stirred at 59°-60°C for 75 hrs., during which time most of the desired coupled product was precipitated. The desired reaction product was isolated quantitatively by flowing a solution of the reaction mixture in aqueous acetic acid through a column of sulfonated polystyrene beads (50-100 mesh). Subsequent elution of the column with aqueous pyridine/water at a 1:10 ratio and evaporation of the eluant under vacuum gave 0.192 g. of the amide (80% yield) as a white solid. Recrystallization from aqueous methanol gave the pure amide (0.109 g.) which was shown to be identical to an authentic sample by TLC analysis.

0098625

CLAIMS

1. A method for preparing alpha-aspartic acid amides, which comprises the steps of:

(a) reacting an alpha-aspartate ester with a beta-hydroxy primary amine in a liquid phase system to form an alpha-aspartic acid amide; and

(b) recovering the amide from the liquid phase system.

2. A method according to Claim 1 wherein the mole ratio of one of the amine and the ester to the other is at least about 2:1.

3. A method according to Claim 2 wherein the concentration of one of the amine and ester is at least about 2 M, the concentration of the other being at least about 1 M.

4. A method according to Claim 2 comprising the further step of removing solvent from the solvent system during said reacting step.

5. A method according to Claim 2 wherein said recovering step comprises precipitating the amide from the solvent system during said reacting step.

6. A method according to Claim 2 wherein the ester is methyl alpha-L-aspartate.

7. A method according to Claim 6 wherein the amine has the formula:

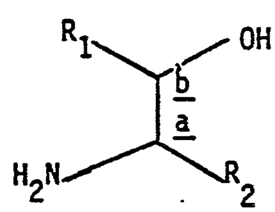

wherein $R_1$ is hydrogen or methyl, wherein $R_2$ is a $C_4$-$C_9$ alkyl group, and wherein carbon atoms a and b have an (S) configuration.

8. A method according to Claim 7 wherein $R_1$ is hydrogen.

9. A method according to Claim 7 wherein $R_1$ is methyl and wherein the amine is in stoichiometric excess.

10. A method according to Claims 8 or 9 wherein $R_2$ is 3-methylbutyl.

0098625

11. A method for preparing alpha-L-aspartic acid amides which comprises the steps of:

(a) providing a solvent system containing an alpha-L-aspartate ester in a concentration of at least about 0.1 M;

(b) adding to the solvent system a beta-hydroxy primary amine in a concentration of at least about 0.2 M and in a moler ratio to the ester of at least about 2:1, the amine having the formula:

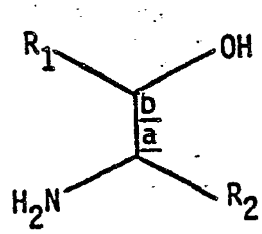

wherein $R_1$ is hydrogen or methyl, wherein $R_2$ is a $C_4$-$C_9$ alkyl group, and wherein carbon atoms a and b have an (S) configuration;

(c) reacting the ester with the amine at a temperature of from about 50° to about 100°C to form an alpha-L-aspartic acid amide;

(d) removing solvent from the solvent system during said reacting step; and

(e) precipitating the amide from the solvent system during said reacting step.

12. A method according to Claim 11 wherein $R_1$ is hydrogen.

13. A method according to Claim 11 wherein $R_1$ is methyl and wherein the temperature during said reacting step does not exceed about 60°C.

14. A method according to Claims 12 or 13 wherein $R_2$ is 3-methylbutyl.

0098625

European Patent Office

# EUROPEAN SEARCH REPORT

Application number

EP 83 20 0775

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | DE-A-2 021 825 (MANZKE) * Page 3; claims * | 1 | C 07 C 103/50 |
| Y | FR-A-2 277 813 (MAGGI) * Example 1 * | 1 | |
| D,Y | EP-A-0 013 044 (THE PROCTER & GAMBLE COMPANY) * Claims * | 1-14 | |
| | | | **TECHNICAL FIELDS SEARCHED (Int. Cl. 3)** |
| | | | C 03 C 103/00<br>C 07 C 102/00 |

The present search report has been drawn up for all claims

| Place of search | Date of completion of the search | Examiner |
|---|---|---|
| THE HAGUE | 08-09-1983 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82